# EUROPÄISCHE PATENTSCHRIFT

(11) **EP 3 275 370 B1**
(45) Veröffentlichungstag und Bekanntmachung des Hinweises auf die Patenterteilung: **28.02.2024**
(21) Anmeldenummer: 17168274.3
(22) Anmeldetag: 26.04.2017
(51) Int. Cl.: A61B 5/18

(54) **VERFAHREN UND VORRICHTUNG ZUM BESTIMMEN EINER DAUER EINES KRITISCHEN ZUSTANDS EINES FAHRERS EINES FAHRZEUGS**
METHOD AND DEVICE FOR DETERMINING A DURATION OF A CRITICAL STATE OF A DRIVER OF A VEHICLE
PROCÉDÉ ET DISPOSITIF DE DÉTERMINATION D'UNE DURÉE D'UN ÉTAT CRITIQUE D'UN CONDUCTEUR D'UN VÉHICULE AUTOMOBILE

(30) Priorität: 26.07.2016 DE 102016213671
(43) Veröffentlichungstag der Anmeldung: 31.01.2018
(73) Patentinhaber: Robert Bosch GmbH, 70442 Stuttgart (DE)
(72) Erfinder: Wulf, Felix, 71636 Ludwigsburg (DE)

(56) Entgegenhaltungen:
- DE-A1- 19 715 519
- DE-A1-102008 056 343

## Beschreibung

### Stand der Technik

Die Erfindung geht aus von einer Vorrichtung oder einem Verfahren nach Gattung der unabhängigen Ansprüche. Gegenstand der vorliegenden Erfindung ist auch ein Computerprogramm.

Ein Fahrzeug kann mit einem Warnsystem zum Warnen eines Fahrers vor Müdigkeit oder Sekundenschlaf ausgestattet sein.

Die DE 10 2008 056 343 A1 offenbart ein Warnsystem für ein Kraftfahrzeug, bei dem auf einen verringerten Aufmerksamkeitsgrad geschlossen wird, wenn eine Gesamtzeitdauer einer Sequenz von mehreren auf die Bedienelemente bezogenen Bedienhandlungen eine Mindestzeitdauer überschreitet.

Die DE 197 15 519 A1 offenbart ein Gerät zur Schätzung des Schläfrigkeitsgrades des Fahrers eines Fahrzeugs. Dabei wird eine Häufigkeitsverteilung der Blinzeldauer des Fahrers berücksichtigt.

### Offenbarung der Erfindung

Vor diesem Hintergrund werden mit dem hier vorgestellten Ansatz ein Verfahren zum Bestimmen einer Dauer eines kritischen Zustands eines Fahrers eines Fahrzeugs, weiterhin eine Vorrichtung, die dieses Verfahren verwendet, sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Es wird ein Verfahren zum Bestimmen einer Dauer eines kritischen Zustands eines Fahrers eines Fahrzeugs vorgestellt, wobei das Verfahren alle Schritte des Anspruchs 1, u.a. folgende Schritte umfasst:
Einlesen eines Zustandssignals, das einen unter Verwendung einer Sensoreinrichtung des Fahrzeugs erfassten Zustand des Fahrers repräsentiert;
Überprüfen, ob das Zustandssignal den kritischen Zustand oder einen nicht kritischen Zustand des Fahrers repräsentiert;
Erhöhen eines die Dauer des kritischen Zustands repräsentierenden Zählerwerts eines Zählers, wenn das Zustandssignal den kritischen Zustand repräsentiert;
Konstanthalten des Zählerwerts, wenn das Zustandssignal den nicht kritischen Zustand repräsentiert; und
Verringern des Zählerwerts, wenn das Zustandssignal nach Ablauf einer Referenzhaltezeit den nicht kritischen Zustand repräsentiert.

Unter einem kritischen Zustand kann ein Zustand des Fahrers verstanden werden, durch den eine Fahrtüchtigkeit des Fahrers beeinträchtigt ist. Erfindungsgemäß handelt es sich bei dem kritischen Zustand um einen Zustand, in dem die Augen des Fahrers für eine längere Dauer, länger als die Dauer eines Lidschlags, geschlossen sind. Dementsprechend kann unter einem nicht kritischen Zustand ein Zustand des Fahrers verstanden werden, in dem die Fahrtüchtigkeit des Fahrers unbeeinträchtigt ist. Das Fahrzeug kann mit einer Sensoreinrichtung zum Überwachen des Fahrers ausgestattet sein. Die Sensoreinrichtung, beispielsweise eine Kamera oder ein Infrarotsensor, kann ausgebildet sein, um einen Kopfbereich des Fahrers, insbesondere eine Augenpartie des Fahrers, zu überwachen. Unter einem Zustandssignal kann ein unter Verwendung der Sensoreinrichtung erzeugtes Signal verstanden werden. Unter einem Zähler kann beispielsweise ein digitales Zählwerk oder ein Softwarezähler verstanden werden. Unter einer Referenzhaltezeit wird eine vorgegebene Höchstdauer verstanden, während der der Zählerwert konstant gehalten werden kann. Die Referenzhaltezeit kann beispielsweise zwischen 0,1 s und 0,5 s liegen.

Der hier vorgestellte Ansatz beruht auf der Erkenntnis, dass ein Zähler zum Bestimmen einer kumulierten Dauer eines kritischen Zustands eines Fahrers verzögert zurückgesetzt werden kann. Dies hat den Vorteil, dass kurze Oszillationen eines den kritischen Zustand repräsentierenden Eingangssignals überbrückt werden können, ohne dass der Zähler jedes Mal zurückgesetzt wird.

Somit kann bei einem Fahrer, der nur kurzzeitig die Augen öffnet und sie danach wieder schließt, frühzeitig ein Sekundenschlaf erkannt werden. Zudem können Unzulänglichkeiten des Eingangssignals überbrückt werden, sodass der Sekundenschlaf auch bei kurzzeitig ausfallendem Eingangssignal erkannt werden kann. Der hier vorgestellte Ansatz ermöglicht somit auch bei kurzzeitigen Sensorfehlern eine effiziente und robuste Erkennung der Dauer des kritischen Zustands.

Gemäß der Erfindung wird im Schritt des Überprüfens überprüft, ob das Zustandssignal einen geschlossenen Zustand der Augen des Fahrers als den kritischen Zustand repräsentiert oder einen geöffneten Zustand der Augen als den nicht kritischen Zustand repräsentiert. Dadurch kann überprüft werden, ob der Fahrer mit geöffneten oder geschlossenen Augen fährt.

Gemäß einer weiteren Ausführungsform kann im Schritt des Erhöhens der Zählerwert weiter erhöht werden, wenn sich beim Überprüfen ergibt, dass das Zustandssignal nachfolgend auf das Konstanthalten oder, zusätzlich oder alternativ, nachfolgend auf das Verringern erneut den kritischen Zustand repräsentiert. Dadurch kann die Dauer des kritischen Zustands kumuliert werden.

Es ist ferner vorteilhaft, wenn im Schritt des Erhöhens der Zählerwert im Wesentlichen linear erhöht wird. Dadurch kann die Dauer des kritischen Zustands mit geringem Rechenaufwand ermittelt werden.

Das Verfahren kann zudem einen Schritt des Ermittelns einer Haltezeit, während der der Zählerwert konstant gehalten wird, umfassen. In einem Schritt des Vergleichens können die Haltezeit und die Referenzhaltezeit miteinander verglichen werden, um eine Abweichung zwischen der Haltezeit und der Referenzhaltezeit zu ermitteln. Im Schritt des Verringerns kann der Zählerwert verringert werden, wenn sich beim Vergleichen ergibt, dass die Haltezeit größer als die Referenzhaltezeit ist. Entsprechend kann im Schritt des Überprüfens das Zustandssignal erneut überprüft werden, wenn sich beim Vergleichen ergibt, dass die Haltezeit kleiner als die Referenzhaltezeit ist. Durch diese Ausführungsform kann die Dauer des kritischen Zustands schnell und mit geringem Speicherbedarf ermittelt werden.

Erfindungsgemäß ist ferner, wenn in einem Schritt des Bestimmens eine Abweichung zwischen dem Zählerwert und einem Grenzwert bestimmt wird. Es muss in einem Schritt des Ausgebens abhängig von der Abweichung zwischen dem Zählerwert und dem Grenzwert ein Warnsignal zum Warnen des Fahrers ausgegeben werden. Unter einem Grenzwert kann beispielsweise ein einen Sekundenschlaf repräsentierender Schwellenwert verstanden werden. Durch diese Ausführungsform kann der Fahrer frühzeitig vor dem kritischen Zustand gewarnt werden.

Gemäß einer weiteren Ausführungsform kann im Schritt des Verringerns der Zählerwert auf null gesetzt werden. Dadurch kann der Zähler einfach zurückgesetzt werden.

Dieses Verfahren kann beispielsweise in Software oder Hardware oder in einer Mischform aus Software und Hardware, beispielsweise in einem Steuergerät, implementiert sein.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung kann die der Erfindung zugrunde liegende Aufgabe schnell und effizient gelöst werden.

Hierzu kann die Vorrichtung zumindest eine Recheneinheit zum Verarbeiten von Signalen oder Daten, zumindest eine Speichereinheit zum Speichern von Signalen oder Daten, zumindest eine Schnittstelle zu einem Sensor oder einem Aktor zum Einlesen von Sensorsignalen von dem Sensor oder zum Ausgeben von Daten- oder Steuersignalen an den Aktor und/oder zumindest eine Kommunikationsschnittstelle zum Einlesen oder Ausgeben von Daten aufweisen, die in ein Kommunikationsprotokoll eingebettet sind. Die Recheneinheit kann beispielsweise ein Signalprozessor, ein Mikrocontroller oder dergleichen sein, wobei die Speichereinheit ein Flash-Speicher, ein EPROM oder eine magnetische Speichereinheit sein kann. Die Kommunikationsschnittstelle kann ausgebildet sein, um Daten drahtlos und/oder leitungsgebunden einzulesen oder auszugeben, wobei eine Kommunikationsschnittstelle, die leitungsgebundene Daten einlesen oder ausgeben kann, diese Daten beispielsweise elektrisch oder optisch aus einer entsprechenden Datenübertragungsleitung einlesen oder in eine entsprechende Datenübertragungsleitung ausgeben kann.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

In einer vorteilhaften Ausgestaltung erfolgt durch die Vorrichtung eine Steuerung des Fahrzeugs. Hierzu kann die Vorrichtung beispielsweise auf Sensorsignale wie Beschleunigungs-, Druck-, Lenkwinkel- oder Umfeldsensorsignale zugreifen. Die Ansteuerung erfolgt über Aktoren wie Brems- oder Lenkaktoren oder ein Motorsteuergerät des Fahrzeugs.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele der Erfindung sind in den Zeichnungen dargestellt und in der nachfolgenden Beschreibung näher erläutert. Es zeigt:
Fig. 1 eine schematische Darstellung eines Fahrzeugs mit einer Vorrichtung gemäß einem Ausführungsbeispiel;
Fig. 2 eine schematische Darstellung einer Vorrichtung gemäß einem Ausführungsbeispiel;
Fig. 3 eine schematische Darstellung eines Verfahrens gemäß einem Ausführungsbeispiel; und
Fig. 4 ein Diagramm zur exemplarischen Darstellung einer kumulierten Dauer eines kritischen Zustands.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

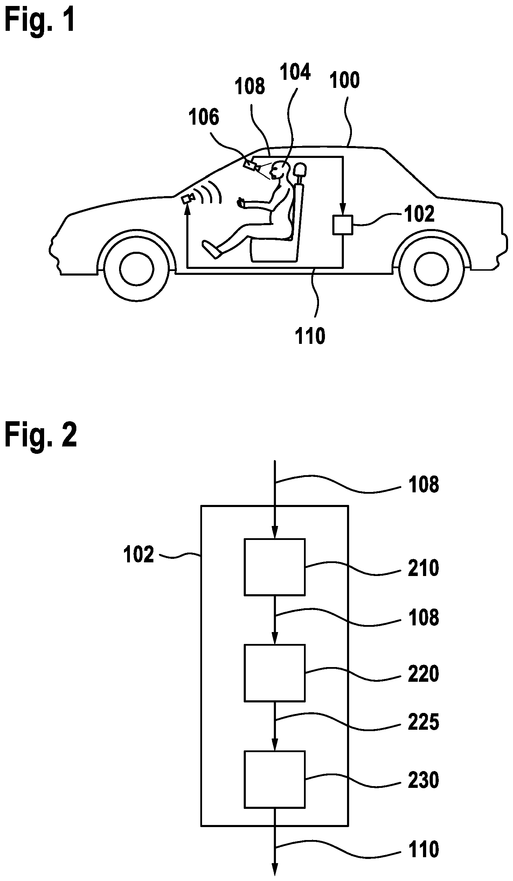

Fig. 1 zeigt eine schematische Darstellung eines Fahrzeugs 100 mit einer Vorrichtung 102 gemäß einem Ausführungsbeispiel. Die Vorrichtung 102 dient zum Bestimmen einer Dauer eines kritischen Zustands eines Fahrers 104 des Fahrzeugs 100. Hierzu ist die Vorrichtung 102 mit einer Sensoreinrichtung 106, hier einer Kamera zum Beobachten eines Gesichts des Fahrers 104, verbunden. Gemäß diesem Ausführungsbeispiel ist die Sensoreinrichtung 106 ausgebildet, um ein einen Zustand des Fahrers 104 repräsentierendes Zustandssignal 108 an die Vorrichtung 102 zu übertragen. Bei dem Zustand handelt es sich etwa um einen geöffneten oder geschlossenen Zustand der Augen des Fahrers 104. Die Vorrichtung 102 ist ausgebildet, um die Dauer des kritischen Zustands, etwa des geschlossenen Zustands der Augen, unter Verwendung des Zustandssignals 108 zu ermitteln. Hierbei kumuliert die Vorrichtung 102 die Dauer des kritischen Zustands mittels eines Zählers, wenn das Zustandssignal 108 den kritischen Zustand repräsentiert. Repräsentiert das Zustandssignal 108 hingegen einen nicht kritischen Zustand des Fahrers, etwa den geöffneten Zustand der Augen, so hält die Vorrichtung 102 die Dauer des kritischen Zustands zunächst konstant. Erst wenn die Dauer des nicht kritischen Zustands eine vorgegebene Referenzhaltezeit tₕ überschreitet, setzt die Vorrichtung 102 den Zähler zurück.

Gemäß dem in Fig. 1 gezeigten Ausführungsbeispiel ist die Vorrichtung 102 ausgebildet, um je nach Dauer des kritischen Zustands ein Warnsignal 110, hier ein akustisches Warnsignal, auszugeben, durch das der Fahrer 104 vor dem kritischen Zustand gewarnt werden kann.

Fig. 2 zeigt eine schematische Darstellung einer Vorrichtung 102 gemäß einem Ausführungsbeispiel, etwa einer Vorrichtung, wie sie vorangehend anhand von Fig. 1 beschrieben ist. Die Vorrichtung 102 umfasst eine Einleseeinheit 210 zum Einlesen des Zustandssignals 108. Ferner umfasst die Vorrichtung 102 eine Zählereinheit 220, kurz Zähler, die ausgebildet ist, um das Zustandssignal 108 von der Einleseeinheit 210 zu empfangen und unter Verwendung des Zustandssignals 108 einen die Dauer des kritischen Zustands repräsentierenden Zählerwert 225 auszugeben. Hierbei ist die Zählereinheit 220 ausgebildet, um zunächst zu überprüfen, ob das Zustandssignal 108 den kritischen Zustand oder den nicht kritischen Zustand des Fahrers repräsentiert. Solange das Zustandssignal 108 den kritischen Zustand repräsentiert, erhöht die Zähluntereinheit 220 den Zählerwert 225. Der Zählerwert 225 repräsentiert somit eine kumulierte Dauer des kritischen Zustands. Sobald die Überprüfung des Zustandssignals 108 ergibt, dass das Zustandssignal 108 den nicht kritischen Zustand repräsentiert, wird die Erhöhung durch die Zählereinheit 220 gestoppt und der Zählerwert 225 konstant gehalten. Überschreitet die Dauer des nicht kritischen Zustands die Referenzhaltezeit tₕ, so setzt die Zählereinheit 220 den Zählerwert 225 auf einen niedrigeren Wert, beispielsweise auf null.

Wenn die Überprüfung des Zustandssignals 108 ergibt, dass das Zustandssignal 108 vor Ablauf der Referenzhaltezeit tₕ wieder den kritischen Zustand repräsentiert, wo wird die Erhöhung des konstant gehaltenen Zählerwerts 225 durch die Zählereinheit 220 fortgesetzt, beispielsweise bis die Überprüfung des Zustandssignals 108 ergibt, dass das Zustandssignal 108 wieder den nicht kritischen Zustand repräsentiert. Somit kann der Zählerwert 225 mit Unterbrechungen immer weiter erhöht werden, bis während einer Phase des den nicht kritischen Zustand anzeigenden Zustandssignal 108 die Referenzhaltezeit tₕ überschritten wird.

Gemäß einem optionalen Ausführungsbeispiel umfasst die Vorrichtung 102 eine Ausgabeeinheit 230, die ausgebildet ist, um den Zählerwert 225 von der Zählereinheit 220 zu empfangen und diesen mit einem Grenzwert, der beispielsweise einen Sekundenschlaf des Fahrers repräsentiert, zu vergleichen. Ergibt der Vergleich, dass der Zählerwert 225 den Grenzwert überschreitet, so gibt die Ausgabeeinheit 230 das Warnsignal 110 zum Warnen des Fahrers aus.

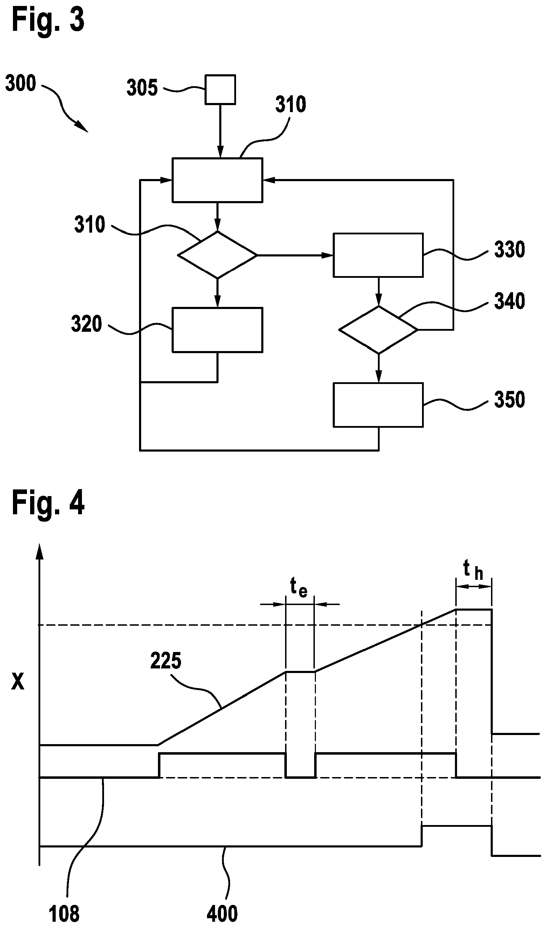

Fig. 3 zeigt ein Ablaufdiagramm eines Verfahrens 300 gemäß einem Ausführungsbeispiel. Das Verfahren 300 kann im Zusammenhang mit einer Vorrichtung, wie sie vorangehend anhand der Figuren 1 und 2 beschrieben ist, durchgeführt werden. Gezeigt ist ein beispielhafter Ablauf einer Rechenvorschrift zur Bestimmung der Dauer des kritischen Zustands. Hierbei wird in einem Schritt 305 das Zustandssignal eingelesen. Anschließend wird in einem Schritt 310 überprüft, ob das Zustandssignal den kritischen Zustand, auch Ereignis genannt, oder den nicht kritischen Zustand repräsentiert. Im Fall eines Ereignisses wird in einem Schritt 320 der Zählerwert des Zählers erhöht, wobei ansprechend auf das Erhöhen im Schritt 310 das Zustandssignal erneut überprüft wird. Andernfalls wird in einem Schritt 330 der Zählerwert konstant gehalten. Hierbei wird beispielsweise eine seit einem letzten Ereignis vergangene Haltezeit tₑ ermittelt, wobei der Zählerwert während der Haltezeit tₑ konstant gehalten wird. In einem Schritt 340 wird die Haltezeit tₑ mit der Referenzhaltezeit tₕ verglichen, um eine Abweichung zwischen der Haltezeit tₑ und der Referenzhaltezeit tₕ zu ermitteln. Ergibt sich hierbei, dass die Haltezeit tₑ größer als die Referenzhaltezeit tₕ ist, so wird in einem Schritt 350 der Zähler zurückgesetzt oder verringert. Ansprechend auf das Zurücksetzen oder Verringern wird der Schritt 310 erneut durchgeführt. Ergibt sich hingegen, dass die Haltezeit tₑ kleiner als die Referenzhaltezeit tₕ ist, so wird der Schritt 310 ohne Zurücksetzen oder Verringern des Zählers direkt wiederholt.

Die einzelnen Schritte des Verfahrens 300 können fortlaufend durchgeführt werden.

Fig. 4 zeigt ein Diagramm zur exemplarischen Darstellung einer kumulierten Dauer eines kritischen Zustands. Die Dauer des kritischen Zustands kann beispielsweise unter Verwendung einer Vorrichtung, wie sie vorangehend anhand der Figuren 1 und 3 beschrieben ist, kumuliert sein. Gezeigt ist eine den Zählerwert 225 repräsentierende Kurve. Unterhalb der Kurve des Zählerwerts 225 ist ein Verlauf des Zustandssignals 108 eingezeichnet, das hier beispielhaft den Verlauf eines Rechtecksignals aufweist. Zu Beginn der Berechnung ist der Zählerwert 225 gleich null. Nach einer kurzen Zeit steigt das Zustandssignal 108 sprungartig auf einen Wert an, der den kritischen Zustand, hier also den geschlossenen Zustand der Augen, repräsentiert. Dabei beginnt der Zählerwert 225 im Wesentlichen linear anzusteigen. Anschließend fällt das Zustandssignal 108 sprungartig auf null ab und bleibt für eine Dauer, die kürzer als die Referenzhaltezeit tₕ ist, auf null. Während dieser Dauer, vorangehend auch Haltezeit tₑ genannt, bleibt der Zählerwert 225 konstant. Anschließend steigt das Zustandssignal 108 wiederum sprungartig auf den den kritischen Zustand repräsentierenden Wert an, sodass sich der Zählerwert 225 wiederum, ausgehend von einem zuletzt ermittelten Wert, im Wesentlichen linear erhöht. Hierbei überschreitet der Zählerwert 225 einen Schwellenwert x, vorangehend auch Grenzwert genannt, was zur Generierung eines Sekundenschlafsignals 400 führt. Ein Verlauf des Sekundenschlafsignals 400, hier ebenfalls ein Rechtecksignal, ist unterhalb des Verlaufs des Zustandssignals 108 eingezeichnet. Der Zählerwert 225 steigt nachfolgend auf das Überschreiten des Schwellenwertes x weiter an, bis das Zustandssignal 108 schließlich wiederum auf null abfällt, sodass der zwischenzeitlich erhöhte Zählerwert 225 konstant bleibt. Die Haltezeit tₑ überschreitet hierbei die Referenzhaltezeit tₕ, was zum Zurücksetzen des Zählers führt. Dementsprechend fällt der Zählerwert 225 sprungartig auf null ab. Somit liegt auch das Sekundenschlafsignal 400 nicht mehr an.

Nachfolgend wird der hier vorgestellte Ansatz anhand der Figuren 1 bis 4 nochmals mit anderen Worten zusammengefasst.

Zunächst erfolgt im Schritt 310 die Erkennung des kritischen Zustands "Augen geschlossen". Anschließend erfolgt die Berechnung der Dauer des kritischen Zustands. Hierbei wird die Zeitdauer berechnet, für die der Zustand "Augen geschlossen" vorliegt. Dies erfolgt mittels eines Zählers, der die Zeit so lange kumuliert, wie die Augen im vorherigen Schritt als geschlossen klassifiziert werden.

Sobald die Augen im Schritt 310 wieder als offen klassifiziert werden, wird der Zähler auf null gesetzt und beginnt beim nächsten Schließen der Augen erneut. Der Wert des Zählers repräsentiert dann die kumulierte Dauer des Zustands "geschlossene Augen".

Hierbei wird nun eine Hysterese eingebaut, die in der Lage ist, kurze Oszillationen des Zustandssignals 108 zu überbrücken, ohne dass der Zähler jedes Mal wieder auf null gesetzt wird. Dies hat den Vorteil, dass bei einem Fahrer, der nur kurzzeitig seine Augen öffnet und sie danach wieder schließt, frühzeitiger ein Sekundenschlaf erkannt werden kann. Zudem können so Unzulänglichkeiten der Eingangssignale überbrückt werden, sodass Sekundenschlaf auch bei kurzzeitig ausfallenden Eingangssignalen erkannt werden kann.

Zur effizienten Berechnung der Dauer des kritischen Zustands wird beispielsweise die in Fig. 3 gezeigte Rechenvorschrift verwendet. Dabei wird der Zähler je nach Vorhandensein des kritischen Zustands, auch Ereignis genannt, entsprechend der zeitlichen Dauer seit einer letzten Prüfung erhöht. Wird diese Rechenvorschrift in einer konstanten und determinierten Zykluszeit durchgeführt, wird hier die Dauer zwischen den Zyklen zum Inkrementieren des Zählers verwendet. Diese Methode hat den Vorteil, dass zur Berechnung der Dauer des kritischen Zustands lediglich ein Speicherelement, nämlich der Zähler, erforderlich ist. Dadurch kann die Dauer sehr effizient berechnet werden.

Es kann passieren, dass der kritische Zustand für kurze Zeiten nicht erkannt wird. Stattdessen kann fälschlicherweise der nicht kritische Zustand, bei dem die Augen geöffnet sind, erkannt werden oder es ist gar keine Erkennung möglich. In diesen Fällen würde der Zähler fälschlicherweise wieder auf null gesetzt, was eine Erkennung längerer kritischer Zustände, etwa eines Sekundenschlafs, unmöglich macht. Zur Lösung dieses Problems wird nun die Referenzhaltezeit tₕ eingeführt, um die Berechnung robust gegenüber kurzzeitigen Sensorfehlern zu machen. Dabei wird der Zustand des Zählers bei Nichtvorhandensein des kritischen Zustands für eine gewisse Zeit gehalten. Erst nach Ablauf der Referenzhaltezeit tₕ wird der Zähler zurückgesetzt oder dekrementiert. Das Ergebnis einer solchen Berechnung ist in Fig. 4 dargestellt. Der Wertebereich der Referenzhaltezeit tₕ wird abhängig von der gegebenen Leistung der Sensoreinrichtung 106 und der erkennenden Dauer der kritischen Zustände gewählt. Es kann beispielsweise tₕ = 0,2 s gesetzt werden.

Die Ausgabe einer Warnentscheidung erfolgt im Wesentlichen nach dem Prinzip, dass gewarnt wird, sobald der Schwellenwert x in der kumulierten Dauer des kritischen Zustands überschritten wird. Beispielsweise erfolgt eine Warnung, wenn die Dauer des kritischen Zustands 1 s erreicht. Der Schwellenwert x ist beispielsweise variabel und wird abhängig von externen Parametern gewählt.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (300) zum Bestimmen einer Dauer eines kritischen Zustands eines Fahrers (104) eines Fahrzeugs (100), wobei das Verfahren (300) folgende Schritte umfasst:
Einlesen (305) eines Zustandssignals (108), das einen unter Verwendung einer Sensoreinrichtung (106) des Fahrzeugs (100) erfassten Zustand des Fahrers (104) repräsentiert;
Überprüfen (310), ob das Zustandssignal (108) den kritischen Zustand oder einen nicht kritischen Zustand des Fahrers (104) repräsentiert, wobei überprüft wird, ob das Zustandssignal (108) einen geschlossenen Zustand der Augen des Fahrers (104), in dem die Augen des Fahrers (104) länger als die Dauer eines Lidschlags geschlossen sind, als den kritischen Zustand repräsentiert, oder einen geöffneten Zustand der Augen als den nicht kritischen Zustand repräsentiert;
Erhöhen (320) eines die Dauer des kritischen Zustands repräsentierenden Zählerwerts (225) eines Zählers, wenn das Zustandssignal (108) den kritischen Zustand repräsentiert;
Konstanthalten (330) des Zählerwerts (225), wenn das Zustandssignal (108) den nicht kritischen Zustand repräsentiert;
Verringern (350) des Zählerwerts (225), wenn das Zustandssignal (108) nach Ablauf einer Referenzhaltezeit (tₕ) den nicht kritischen Zustand repräsentiert, wobei die Referenzhaltezeit (tₕ) eine vorgegebene Höchstdauer ist, während der der Zählerwert (225) konstant gehalten wird; und
Bestimmen einer Abweichung zwischen dem Zählerwert (225) und
einem Grenzwert (x), wobei in einem Schritt des Ausgebens abhängig von der Abweichung zwischen dem Zählerwert (225) und dem Grenzwert (x) ein Warnsignal (110) zum Warnen des Fahrers (104) ausgegeben wird.

2. Verfahren (300) gemäß Anspruch 1, bei dem im Schritt des Erhöhens (320) der Zählerwert (225) weiter erhöht wird, wenn sich beim erneuten Überprüfen (310) ergibt, dass das Zustandssignal (108) nachfolgend auf das Konstanthalten (330) und/oder das Verringern (350) erneut den kritischen Zustand repräsentiert.

3. Verfahren (300) gemäß einem der vorangegangenen Ansprüche, bei dem im Schritt des Erhöhens (320) der Zählerwert (225) linear erhöht wird.

4. Verfahren (300) gemäß einem der vorangegangenen Ansprüche, mit einem Schritt des Ermittelns einer Haltezeit (tₑ), während der der Zählerwert (225) konstant gehalten wird, wobei in einem Schritt des Vergleichens (340) die Haltezeit (tₑ) und die Referenzhaltezeit (tₕ) miteinander verglichen werden, um eine Abweichung zwischen der Haltezeit (tₑ) und der Referenzhaltezeit (tₕ) zu ermitteln, wobei im Schritt des Verringerns (350) der Zählerwert (225) verringert wird, wenn sich beim Vergleichen (340) ergibt, dass die Haltezeit (tₑ) größer als die Referenzhaltezeit (tₕ) ist, wobei beim erneuten Überprüfen (310) das Zustandssignal (108) erneut überprüft wird, wenn sich beim Vergleichen (340) ergibt, dass die Haltezeit (tₑ) kleiner als die Referenzhaltezeit (tₕ) ist.

5. Verfahren (300) gemäß einem der vorangegangenen Ansprüche, bei dem im Schritt des Verringerns (350) der Zählerwert (225) auf null gesetzt wird.

6. Vorrichtung (102) mit einer Einleseeinheit (210), einem Zähler (220), einer Recheneinheit, sowie einer Ausgabeeinheit (230), die ausgebildet sind, um das Verfahren (300) gemäß einem der vorangegangenen Ansprüche auszuführen.

7. Computerprogrammprodukt, umfassend Befehle, die bei der Ausführung des Programms durch einen Computer diesen veranlassen, das Verfahren (300) gemäß einem der Ansprüche 1 bis 5 auszuführen.

8. Maschinenlesbares Speichermedium, auf dem das Computerprogrammprodukt nach Anspruch 7 gespeichert ist.

## Claims

1. Method (300) for determining a duration of a critical state of a driver (104) of a vehicle (100), wherein the method (300) comprises the following steps:
reading in (305) a state signal (108) representing a state of the driver (104) detected using a sensor device (106) of the vehicle (100);
checking (310) whether the state signal (108) represents the critical state or a non-critical state of the driver (104), wherein a check is carried out in order to determine whether the state signal (108) represents a closed state of the eyes of the driver (104), in which the eyes of the driver (104) are closed for longer than the duration of a blink, as the critical state, or represents an open state of the eyes as the non-critical state;
incrementing (320) a counter value (225)of a counter that represents the duration of the critical state if the state signal (108) represents the critical state;
keeping (330) the counter value (225) constant if the state signal represents the non-critical state;
decrementing (350) the counter value (225) if the state signal (108) represents the non-critical state after expiry of a reference holding time (tₕ), wherein the reference holding time (tₕ) is a predefined maximum duration, during which the counter value (225) is kept constant; and
determining a discrepancy between the counter value (225) and a limit value (x), wherein, in an outputting step, a warning signal (110) for warning the driver (104) is output depending on the discrepancy between the counter value (225) and the limit value (x).

2. Method (300) according to Claim 1, in which, in the incrementing step (320), the counter value (225) is incremented further if the renewed checking (310) reveals that the state signal (108) represents the critical state again after the counter value has been kept constant (330) and/or has been decremented (350).

3. Method (300) according to one of the preceding claims, in which the counter value (225) is increased linearly in the incrementing step (320).

4. Method (300) according to one of the preceding claims, having a step of determining a holding time (tₑ), during which the counter value (225) is kept constant, wherein the holding time (tₑ) and the reference holding time (tₕ) are compared with one another in a comparison step (340) in order to determine a discrepancy between the holding time (tₑ) and the reference holding time (tₕ), wherein, in the decrementing step (350), the counter value (225) is decremented if the comparison (340) reveals that the holding time (tₑ) is greater than the reference holding time (tₕ), wherein, during the renewed checking (310), the state signal (108) is checked again if the comparison (340) reveals that the holding time (tₑ) is less than the reference holding time (tₕ) .

5. Method (300) according to one of the preceding claims, in which the counter value (225) is set to zero in the decrementing step (350).

6. Apparatus (102) having a reading-in unit (210), a counter (220), a computing unit, and an output unit (230), which are designed to carry out the method (300) according to one of the preceding claims.

7. Computer program product comprising instructions that, when the program is executed by a computer, cause said computer to execute the method (300) according to one of Claims 1 to 5.

8. Machine-readable storage medium on which the computer program product according to Claim 7 is stored.

## Revendications

1. Procédé (300) de détermination d'une durée d'un état critique d'un conducteur (104) d'un véhicule (100), le procédé (300) comprenant les étapes suivantes :
lecture (305) d'un signal d'état (108), lequel représente un état du conducteur (104) détecté en utilisant un dispositif capteur (106) du véhicule (100) ;
vérification (310) si le signal d'état (108) représente l'état critique ou un état non critique du conducteur (104), un contrôle étant effectué afin de vérifier si le signal d'état (108) représente un état fermé des yeux du conducteur (104), dans lequel les yeux du conducteur (104) sont fermés plus longtemps que la durée d'un battement de paupière, en tant que l'état critique, ou un état ouvert des yeux en tant que l'état non critique ;
augmentation (320) d'une valeur de compteur (225) d'un compteur qui représente la durée de l'état critique lorsque le signal d'état (108) représente l'état critique ;
maintien de manière constante (330) de la valeur de compteur (225) lorsque le signal d'état (108) représente l'état non critique ;
réduction (350) de la valeur de compteur (225) lorsque le signal d'état (108) représente l'état non critique après l'écoulement d'un temps de maintien de référence (tₕ), le temps de maintien de référence (tₕ) étant une durée maximale prédéterminée pendant laquelle la valeur de compteur (225) est maintenue constante ; et
détermination d'un écart entre la valeur de compteur (225) et une valeur limite (x), un signal d'avertissement (110) destiné à avertir le conducteur (104) étant délivré dans une étape de délivrance, en fonction de l'écart entre la valeur de compteur (225) et la valeur limite (x) .

2. Procédé (300) selon la revendication 1, dans lequel, à l'étape d'augmentation (320), la valeur de compteur (225) est augmentée davantage si, lorsqu'il résulte d'une nouvelle vérification (310) que le signal d'état (108) représente de nouveau l'état critique à la suite du maintien constant (330) et/ou de la réduction (350).

3. Procédé (300) selon l'une des revendications précédentes, dans lequel, à l'étape d'augmentation (320), la valeur de compteur (225) est augmentée linéairement.

4. Procédé (300) selon l'une des revendications précédentes, comprenant une étape d'identification d'un temps de maintien (tₑ) pendant laquelle la valeur de compteur (225) est maintenue constante, le temps de maintien (tₑ) et le temps de maintien de référence (tₕ) étant comparés l'un à l'autre dans une étape de comparaison (340) afin d'identifier un écart entre le temps de maintien (tₑ) et le temps de maintien de référence (tₕ), la valeur de compteur (225) étant réduite dans l'étape de réduction (350) lorsqu'il résulte de la comparaison (340) que le temps de maintien (tₑ) est supérieur au temps de maintien de référence (tₕ), le signal d'état (108) étant de nouveau vérifié lors de la nouvelle vérification (310) lorsqu'il résulte de la comparaison (340) que le temps de maintien (tₑ) est inférieur au temps de maintien de référence (tₕ).

5. Procédé (300) selon l'une des revendications précédentes, dans lequel, à l'étape de réduction (350), la valeur de compteur (225) est mise à zéro.

6. Agencement (102) comprenant une unité de lecture (210), un compteur (220), une unité de calcul ainsi qu'une unité de sortie (230), qui sont configurés pour mettre en œuvre le procédé (300) selon l'une des revendications précédentes.

7. Produit de programme informatique, comprenant des instructions qui, lors de l'exécution du programme par un ordinateur, amènent celui-ci à mettre en œuvre le procédé (300) selon l'une des revendications 1 à 5.

8. Support de stockage lisible par machine, sur lequel est stocké le produit de programme informatique selon la revendication 7.
